(19) Europäisches Patentamt
European Patent Office
Office européen des brevets

(11) Publication number: 0 001 319
A1

# (12) EUROPEAN PATENT APPLICATION

(21) Application number: 78300214.0

(22) Date of filing: 31.07.78

(51) Int. Cl.[2]: C 07 D 233/78
C 07 C 101/72, C 12 D 13/06
//C07D499/68

(30) Priority: 18.08.77 GB 34803/77

(43) Date of publication of application: 04.04.79 Bulletin 79/7

(84) Designated contracting states: BE CH DE FR GB NL SE

(71) Applicant: BEECHAM GROUP LIMITED
Beecham House Great West Road
Brentford, Middlesex(GB)

(72) Inventor: Dales, John Robert Mansfield
20 The Hooe
Littlehampton Sussex(GB)

(74) Representative: Hesketh, Alan, Dr. et al,
Beecham Pharmaceuticals Yew Tree Bottom Road
Epsom, Surrey, KT18 5XQ(GB)

## (54) Process for the preparation of hydroxyphenyl hydantoin and of hydroxyphenyl glycine, and compounds thus obtained.

(57) 5-(p-Hydroxyphenyl) hydantoin of formula (I):

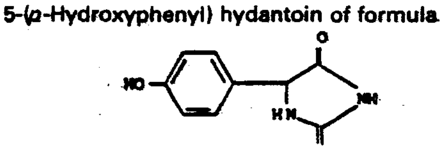

(I)

is prepared by reacting phenol, glyoxylic acid and urea in the presence of an acid catalyst in an aqueous medium.

The hydantoin may be hydrolysed to produce the amino acid, p-hydroxyphenylglycine, preferably to the D-amino acid, either by selective enzymatic hydrolysis and subsequent chemical hydrolysis, or by alkaline hydrolysis and subsequent resolution.

EP 0 001 319 A1

Croydon Printing Company Ltd

TITLE MODIFIED
see front page



Process for the Preparation of Hydroxyphenylhydantoin

This invention relates to a process for the preparation of 5-(p-hydroxyphenyl)hydantoin, which is a usefu intermediate in the preparation of p-hydroxyphenylglyci For example, British Patent No. 1,506,067 describes the preparation of D(-)-N-carbamoyl-p-hydroxyphenylglycine the corresponding amino acid, D(-)-p-hydroxyphenylglyci by selective enzymatic hydrolysis of D,L-5-(p-hydroxyp hydantoin. The D(-)-p-hydroxyphenylglycine is useful an intermediate for the preparation of the penicillin amoxycillin.

5-(p-Hydroxyphenyl)hydantoin is known from Harvi and Herbst,

J. Org. Chem. Vol. 19, 1944, pp. 21 - 30.,

as being prepared by heating p-hydroxybenzaldehyde, potassium cyanide and ammonium carbonate in aqueous al holic solution. The disadvantage of this method for a commercial process is the availability and cost of the starting material, p-hydroxybenzaldehyde. The present invention relates to a process which employs readily a lable and economical starting materials.

According to the present invention there is prov a process for the preparation of 5-(p-hydroxyphenyl)-

BAD ORIGINAL

hydantoin of formula (I):

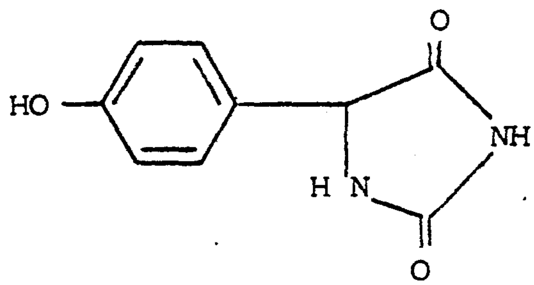

(I)

which process comprises reacting phenol with glyoxylic acid and urea in the presence of an acid catalyst in an aqueous medium.

Although these three reagents have been used in various combinations in the past, the prior art does not suggest that they can be used to produce compound (I) above. In fact, the prior art teaches away from such a process.

For example, Ben-Ishai et al [Tetrahedron, Vol.33, 1191 (1977)] discloses that, whereas reaction of an N-substituted urea, $NH_2.CO.NHCH_3$, with glyoxylic acid can give a hydantoin of formula (II):

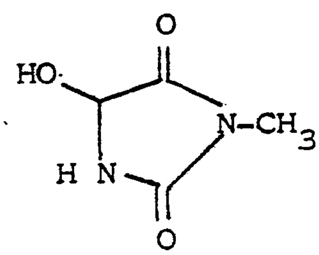

(II) ,

reaction of glyoxylic acid (or a derivative) with urea itself in methanol gives a non-cyclic compound of formula (III):

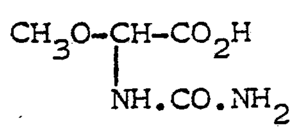

(III) .

British Patent No. 1,435,268, in the name of Smithkline Corporation, discloses <u>inter</u> <u>alia</u> the pre- preparation of N-carbamyl-2-(p-hydroxyphenyl)glycine of formula (IV) by reacting phenol with glyoxylic acid and urea, without solvent.

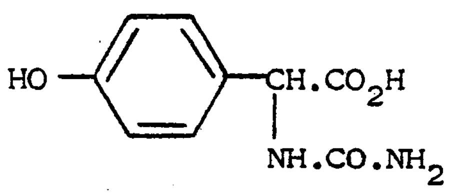

(IV)

Ben Ishai et al [Tetrahedron, Vol. 33, 3261 (1977)] also describes the preparation of a substituted N-carbamyl-p-hydroxyphenylglycine of formula (V) by reaction of phenol with a substituted urea-glyoxylic acid adduct of formula (VI):

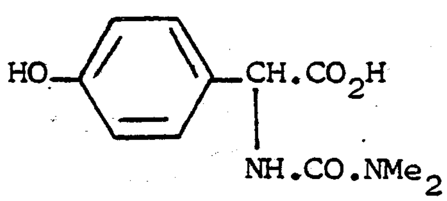

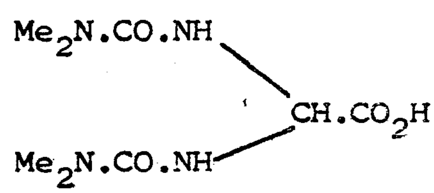

(V) (VI)

Neither this reference nor the Smithkline patent suggests any formation of hydantoins. It is, therefore, surprising that the process of the present invention, using the same three reagents as the Smithkline patent but in aqueous media and in the presence of acid, produces the hydantoin of formula (I).

Suitable acid catalysts for this process include sulphuric acid, hydrochloric acid and hydrobromic acid, of which we have found hydrochloric acid to be the most suitable.

The reaction is preferably carried out in water but water-miscible polar organic solvents, such as acetic acid, may be added to the reaction medium.

The reaction should be carried out at a temperature in excess of 25°C and up to 100°C. A suitable temperature range for this reaction is from 30°-50°C and a particularly convenient temperature is from 35°-45°C.

The time for which the reaction is allowed to proceed is dependent on the quantity of catalyst used and the temperature at which the reaction is performed. About 20 hours is convenient.

It is possible to carry out the process of this invention in two stages, for example, by reacting urea with glyoxylic acid first and then adding phenol.

It is preferable that the phenol is in excess in the reaction, a suitable ratio being approximately 2 moles of phenol to 1 mole of glyoxylic acid. The urea should also be present in a proportion from 1 to 1.5 mole per 1 mole of glyoxylic acid.

Normally, the hydantoin of formula (I) is precipitated during the reaction and can be filtered off. After filtration, the hydantoin may be washed or reslurried with water to remove excess phenol. In the product, when the reaction is carried out using hydrochloric acid as catalyst, there are no detectable quantities of the N-carbamyl-hydroxyphenylglycine of formula (IV) above.

The hydantoin of formula (I) may be hydrolysed to produce p-hydroxyphenylglycine. One method comprises selective enzymatic hydrolysis to produce D-N-carbamyl-p-hydroxyphenylglycine and subsequent hydrolysis to D-(-)-p-hydroxyphenylglycine, for example, as described in British Patent No. 1,506,067.

Alternatively, the hydantoin (I) may be hydrolysed to prepare DL-p-hydroxyphenylglycine, which may be resolved by known methods to yield the D-amino acid. One such resolution method is disclosed in British Patent No. 1,318,216 and involves the use of O,N-diacetyl-p-hydroxyphenylglycine.

The hydrolysis of the hydantoin (I) may be carried out in the presence of acid or alkali. An alkaline hydrolysis is preferred. Suitable bases for the hydrolysis reaction include alkali metal hydroxides such as sodium and potassium hydroxide. Preferably the hydrolysis is carried out in water. The temperature at which the hydrolysis reaction is carried out should be at about the reflux temperature of the reaction medium.

p-Hydroxyphenylglycine prepared in this way may be isolated directly from the reaction medium by known methods and acetylated if required. Alternatively, it may be converted _in situ_ into O,N-diacetyl-p-hydroxyphenylglycine. Excess of base in the reaction medium is first neutralised and an acetylating agent is then added. Suitable acetylating agents include acetic anhydride and acetyl chloride.

The following examples illustrate the process of this invention.

EXAMPLE 1

Preparation of 5-(p-hydroxyphenyl)hydantoin

A mixture of aqueous glyoxylic acid (26.7 ml, 0.27 mole), urea (16.2 g, 0.27 mole), phenol (50.7 g, 0.54 mole), concentrated sulphuric acid (4 ml) and water (50 ml) was stirred and heated at 50°C for 2 days. The reaction mixture was cooled to room temperature and stirred for 30 minutes. The solid which had formed was filtered off, washed with water (25 ml) and dried in a fan oven at 40°C (11.0 g, m.p. 260°C decomp.). The product was recrystallised from methanol (m.p. 272°C decomp.).

The IR spectrum of the product was identical with the spectrum of authentic p-hydroxyphenylhydantoin.

EXAMPLE 2

Preparation of O,N-diacetyl-p-hydroxyphenylglycine

A mixture of aqueous glyoxylic acid (26.7 ml, 0.27 mole), urea (18 g, 0.3 mole), phenol (50 g, 0.53 mole) and concentrated hydrochloric acid (50 ml, S.G. 1.18) was stirred and heated at 40°C for 20 hours. The reaction mixture was cooled to room temperature and the solid filtered off. The filter cake was washed with water (20 mls) and then reslurried in water (100 ml) at 40°C for 30 minutes. The product was filtered off, washed with water (25 mls) and dried in a fan oven at 40°C to yield an off-white powder (23.5 g, m.p. 211-212°C).

The product (23.5 g) was heated under reflux in 20% w/w sodium hydroxide solution (235 g) for 21 hours. During the last 2 hours of the reflux, air was bubbled through the reaction mixture. The mixture was cooled to room temperature and water (40 ml) was added with stirring. The pH of the solution was adjusted to 8 with concentrated hydrochloric

acid and then acetic anhydride (18 ml) was added. During the addition the temperature was maintained at 20°C with cooling and the pH at 8 by suitable addition of 50% w/v sodium hydroxide solution. After the acetic anhydride had been added the mixture was stirred at pH 8 for 1 hour and then the pH was lowered to 1 with concentrated hydrochloric acid. The mixture was stirred for 1 hour at 10°C and the product filtered off, washed with water (30 ml) and dried in the fan oven at 40°C to yield an off-white powder (23 g, m.p. 207°C) Eq. Wt. 248.

The IR spectrum of the product was identical to that obtained from an authentic sample of O,N-diacetyl-p-hydroxyphenylglycine.

EXAMPLE 3

Preparation of p-hydroxyphenylglycine

A mixture of aqueous glyoxylic acid (26.7 ml, 0.27 mole), urea (20 g, 0.33 mole), phenol (45 g, 0.48 mole) and 48% hydrobromic acid (50 ml) was stirred and heated at 40°C for 18 hours. The reaction mixture was cooled to room temperature and the product filtered off. The product was washed with water (20 ml) and then reslurried in water (100 ml) at 40°C for 1 hour. The product was filtered off and washed with water (3x25 ml) and dried in a fan oven at 40°C (13.6 g, m.p. 263-265°C).

The product (13.6 g) was hydrolysed according to the method given in example 2. After cooling to room temperature, water (40 ml) was added to the stirred reaction mixture and the pH adjusted to 4.6 with concentrated hydrochloric acid. The reaction mixture was stirred for 1 hour at 10°C and the product was filtered off, washed with water (10 ml)

and then with acetone (25 ml). The white crystalline product was dried in a fan oven at 40°C (8.8 g, m.p. 220-223°C).

The IR spectrum of the product was identical with that obtained from a sample of authentic p-hydroxyphenylglycine.

EXAMPLE 4

Preparation of 5-(p-hydroxyphenyl)hydantoin

A mixture of aqueous glyoxylic acid (26.7 ml, 0.27 mole), urea (20 g, 0.33 mole), phenol (45 g, 0.48 mole) and concentrated hydrochloric acid (50 ml, S.G. 1.18) was stirred at room temperature for 3 hours and then at 50°C for 20 hours. The reaction mixture was cooled to room temperature and the product filtered off. The product was reslurried in water (150 ml) at 40°C for 1 hour and then filtered off, washed with water (3x25 ml) and dried in a fan oven at 40°C (23.2 g, m.p. 234-235°C).

EXAMPLE 5

Preparation of 5-(p-hydroxyphenyl)hydantoin

A mixture of aqueous glyoxylic acid (26.7 ml, 0.27 mole), urea (16.2 g, 0.27 mole), phenol (50 g, 0.53 mole) and concentrated hydrochloric acid (75 ml, S.G. 1.8) was stirred and heated at 40°C for 20 hours. The reaction mixture was cooled to room temperature, the product filtered off and washed with water (10 ml). The product was reslurried in water (100 ml) at 40°C for 30 minutes, filtered off and washed with water (25 ml) and dried in a fan oven at 40°C (21.6 g, m.p. 243-245°C).

## CLAIMS

1. A process for the preparation of 5-(p-hydroxyphenyl)-hydantoin of formula (I):

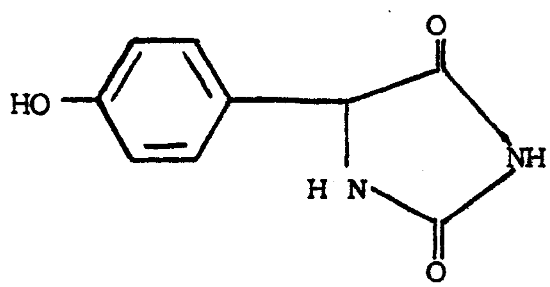

(I)

which process comprises reacting phenol with glyoxylic acid and urea in the presence of an acid catalyst in an aqueous medium.

2. A process as claimed in claim 1 wherein the acid catalyst is hydrochloric acid.

3. A process as claimed in either claim 1 or claim 2 wherein an excess of phenol is employed.

4. A process as claimed in claim 1 wherein the hydantoin of formula (I) is subsequently hydrolysed to produce p-hydroxyphenylglycine.

5. A process as claimed in claim 4 which comprises selective enzymatic hydrolysis to produce D-N-carbamyl-p-hydroxyphenylglycine; and subsequent hydrolysis to D-(-)-p-hydroxyphenylglycine.

6. A process as claimed in claim 4 wherein the hydantoin of formula (I) is hydrolysed in the presence of a base to give DL-p-hydroxyphenylglycine; and optionally subsequently acetylated to produce D,L-O,N-diacetyl-p-hydroxyphenylglycine.

7. A process as claimed in claim 1 substantially as described in any one of examples 1 to 5.

8. 5-(p-Hydroxyphenyl)hydantoin whenever prepared by a process as claimed in any one of claims 1 to 3 or 7.

9. p-Hydroxyphenylglycine whenever prepared by a process as claimed in any one of claims 4 to 6.

0001319

Application number

European Patent Office

# EUROPEAN SEARCH REPORT

EP 78 300 214.0

| DOCUMENTS CONSIDERED TO BE RELEVANT | | | CLASSIFICATION OF THE APPLICATION (Int. Cl.²) |
|---|---|---|---|
| Category | Citation of document with indication, where appropriate, of relevant passages | Relevant to claim | |
| X | DE - A - 2 621 076 (SNAMPROGETTI)<br>* claims 1, 2 *<br>-- | 4,5,7,9 | C 07 D 233/78<br>C 07 C 101/72<br>C 12 D 13/06//<br>C 07 D 499/68 |
| - | DE - A - 2 631 048 (SNAMPROGETTI)<br>* claim 1 *<br>-- | 4,5,7,9 | |
| - | US - A - 3 668 221 (SUMITOMO)<br>* claim 1 *<br>-- | 4,6,7 | TECHNICAL FIELDS SEARCHED (Int.Cl.²) |
| - | US - A - 3 790 599 (ZUNDEL)<br>* claim 1 *<br>-- | 4,6,7 | C 07 D 233/78<br>C 07 C 101/72<br>C 07 D 499/68<br>C 12 D 13/06 |
| A | DE - A - 2 134 251 (STERWIN)<br>-- | | |
| D,A | TETRAHEDRON vol. 33, May 1977, Exeter, BEN-ISHAI et al "The reactions of ureas with glyoxylic acid and methyl glyoxalate" page 1191 to 1196<br>-- | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| D,P,A | TETRAHEDRON vol. 33, December 1977, Exeter, BEN-ISHAI et al "Ureido-alkylations and oxyalkylation of aromatic compounds with glyoxylic acid derivatives" page 3261 to 3264<br>-- | | &: member of the same patent family, corresponding document |

X The present search report has been drawn up for all claims

| Place of search | Date of completion of the search | Examiner |
|---|---|---|
| Berlin | 03-11-1978 | FROELICH |

EPO Form 1503.1 06.78

0001319

European Patent Office

# EUROPEAN SEARCH REPORT

Application number
EP 78 300 214.0



| DOCUMENTS CONSIDERED TO BE RELEVANT | | | CLASSIFICATION OF THE APPLICATION (Int. Cl.²) |
|---|---|---|---|
| Category | Citation of document with indication, where appropriate, of relevant passages | Relevant to claim | |
| A | CH - A - 566 965 (BEECHAM)<br>-- | | |
| D | GB - A - 1 435 268 (SMITHKLINE)<br>* claims 1, 2 *<br>-- | 1-3 | |
| E | NL - A - 78 02960 (KANEGAFUCHI)<br>& BE 865 027<br>* Specification *<br>---- | 1-3,7,8 | TECHNICAL FIELDS SEARCHED (Int. Cl.²) |